# EUROPEAN PATENT SPECIFICATION

(11) **EP 4 400 074 B1**
(45) Date of publication and mention of the grant of the patent: **24.12.2025**
(21) Application number: 22867723.3
(22) Date of filing: 08.09.2022
(51) Int. Cl.: A61C 13/00, A61C 9/00, A61B 5/00, A61C 13/08, A61C 5/70, G06T 1/00, G06T 19/20, G16H 50/50, G16H 30/00, G06T 17/00

(54) **METHOD FOR PROCESSING INTRAORAL IMAGE AND DATA PROCESSING DEVICE**
VERFAHREN ZUR VERARBEITUNG EINES INTRAORALEN BILDES UND DATENVERARBEITUNGSVORRICHTUNG
PROCÉDÉ DE TRAITEMENT D'IMAGE INTRABUCCALE ET DISPOSITIF DE TRAITEMENT DE DONNÉES

(30) Priority: 09.09.2021 KR 20210120535; 08.10.2021 KR 20210134465; 07.09.2022 KR 20220113778
(43) Date of publication of application: 17.07.2024
(73) Proprietor: Medit Corp., Seoul 07207 (KR)
(72) Inventor: LEE, Sung Hoon, Seoul 07207 (KR); ROSLYAKOVA, Maria, Seoul 07207 (KR)
(74) Representative: Kim Kang, Jae Hee
(86) International application number: PCT/KR2022/013518
(87) International publication number: WO 2023/038455

(56) References cited:
- KR-A- 20190 074 062
- KR-A- 20210 084 821
- KR-B1- 101 888 361
- KR-B1- 102 138 921
- US-A1- 2008 261 165
- US-A1- 2017 273 763
- US-A1- 2018 028 294
- US-A1- 2021 244 518

## Description

### TECHNICAL FIELD

The disclosure relates to a method of processing an intraoral image and a data processing apparatus. Specifically, disclosed embodiments relate to a method and apparatus for processing an intraoral image, by which a prosthesis target tooth may be automatically recognized and selected based on the intraoral image.

### BACKGROUND ART

Dental Computer Aided Design/Computer Aided Manufacturing (CAD/CAM) technology is widely used for dental treatment, particularly, prosthetic treatment. The most important thing in dental treatment using CAD/CAM is to acquire precise three-dimensional data on the shape of an object, such as a patient's teeth, gums, and jawbone. When performing dental treatment, accurate calculation may be performed by a computer by using three-dimensional data obtained from an object. For example, to acquire three-dimensional data of an object in a dental CAD/CAM treatment process, methods such as computed tomography (CT), magnetic resonance imaging (MRI), and optical scanning may be used.

When designing a prosthesis (a crown) in a CAD program, a user basically needs to 'select' a tooth needed for design from data scanned from a patient's oral cavity. In other words, when designing a prosthesis, the outer surface of a prosthesis to be designed is based on a pre-preparation tooth and the inner surface of the prosthesis is based on a preparation tooth to make a basic shape of the prosthesis, and then the basic shape of the prosthesis is processed in detail to make a final prosthesis. Therefore, basic data required for design, a pre-preparation tooth surface and a preparation tooth surface, have to be selected. A simple way to design a prosthesis without a tooth selection process is required.

US2018/028294 discloses a method for recognizing dental information for generating dental restoration models, involving identifying dental features in patient's scan data based on output probability values of deep neural network using trained deep neural network.

### DESCRIPTION OF EMBODIMENTS

### TECHNICAL PROBLEMS

The invention is as defined in the appended claims.

Disclosed embodiments provide a method and apparatus for processing an intraoral image, by which a prosthesis target tooth is automatically recognized and selected from the intraoral image without a user having to directly select the prosthesis target tooth.

### SOLUTION TO PROBLEM

According to an embodiment, a method of processing an intraoral image includes obtaining a first intraoral image including pre-preparation tooth data and a second intraoral image including preparation tooth data, obtaining a margin line based on the preparation tooth data, and automatically recognizing and selecting a prosthesis target tooth from at least one of the first intraoral image and the second intraoral image based on the margin line.

According to an embodiment, the obtaining of the margin line based on the preparation tooth data may include obtaining the margin line by automatically recognizing the margin line based on the preparation tooth data or providing a user interface, which allows setting the margin line, and receiving a user input for setting the margin line through the user interface.

According to an embodiment, the automatically recognizing and selecting of the prosthesis target tooth from the first intraoral image based on the margin line may include obtaining an intersection point where one or more normal vectors obtained based on the margin line intersect with the pre-preparation tooth data, and identifying a tooth area of the prosthesis target tooth by using a curvature value of the obtained intersection point and a curvature value of points included in the pre-preparation tooth data.

According to an embodiment, the automatically recognizing and selecting of the prosthesis target tooth from the first intraoral image based on the margin line may include obtaining a predefined figure surrounding the pre-preparation tooth data by using the margin line, and automatically recognizing the prosthesis target tooth by selecting the pre-preparation tooth data that falls within the predefined figure.

According to an embodiment, the automatically recognizing and selecting of the prosthesis target tooth from the second intraoral image based on the margin line may include selecting the prosthesis target tooth by cutting the preparation tooth data by using the margin line.

According to an embodiment, the method may further include generating an outer surface of a prosthesis model to restore the prosthesis target tooth by using the prosthesis target tooth automatically recognized from the first intraoral image based on the margin line, generating an inner surface of the prosthesis model by using the prosthesis target tooth automatically recognized from the second intraoral image based on the margin line, and generating the prosthesis model by using the outer surface of the prosthesis model and the inner surface of the prosthesis model.

According to an embodiment, the generating of the outer surface of the prosthesis model may include obtaining an initial outer surface from the pre-preparation tooth data by using the margin line, generating a margin loop mesh based on the margin line, generating a closed tooth by connecting the initial outer surface to the margin loop mesh, and generating the outer surface of the prosthesis model by removing a predefined area from the closed tooth.

According to an embodiment, the generating of the inner surface of the prosthesis model may include obtaining an initial inner surface by cutting the preparation tooth data by using the margin line, identifying a cement area and a non-cement area in the initial inner surface, obtaining an offset cement area by offsetting the cement area by a predetermined distance, and generating the inner surface of the prosthesis model by connecting the offset cement area to the non-cement area.

According to an embodiment, a data processing apparatus for processing an intraoral image includes a memory including one or more instructions, and a processor configured to, by executing the one or more instructions, obtain a first intraoral image including pre-preparation tooth data and a second intraoral image including preparation tooth data, obtain a margin line based on the preparation tooth data, and automatically recognize and select a prosthesis target tooth from at least one of the first intraoral image and the second intraoral image based on the margin line.

According to an embodiment, there is provided a computer-readable recording medium on which a program implemented to perform an intraoral image processing method by a computer is recorded, wherein the intraoral image processing method includes obtaining a first intraoral image including pre-preparation tooth data and a second intraoral image including preparation tooth data, obtaining a margin line based on the preparation tooth data, and automatically recognizing and selecting a prosthesis target tooth from at least one of the first intraoral image and the second intraoral image based on the margin line.

### ADVANTAGEOUS EFFECTS OF DISCLOSURE

According to the method and apparatus for processing an intraoral image, according to the disclosed embodiment, it is possible to automatically recognize and select a prosthesis target tooth by using a margin line without a user's manual process of selecting a prosthesis target tooth.

According to the method and apparatus for processing an intraoral image, according to the disclosed embodiment, a prosthesis for a prosthesis target tooth may be made without a user's manual selection of a prosthesis target tooth.

### BRIEF DESCRIPTION OF DRAWINGS

The present disclosure may be readily understood with a combination of the following detailed descriptions and the accompanying drawings, wherein reference numbers refer to structural elements.
FIG. 1 is a reference diagram for explaining the creation of a prosthesis through tooth preparation, according to embodiments.
FIG. 2 is a diagram for explaining an intraoral image processing system according to an embodiment.
FIG. 3 is a block diagram illustrating a data processing apparatus according to an embodiment.
FIG. 4 is a flowchart illustrating a method of processing an intraoral image in a data processing apparatus according to an embodiment.
FIG. 5 illustrates an example of programs that generate a prosthesis model in the data processing apparatus 100, according to an embodiment.
FIG. 6 is a reference diagram for explaining an example of automatically recognizing and displaying a prosthesis target tooth in a first intraoral image or a second intraoral image, according to an embodiment.
FIG. 7 is a flowchart illustrating the process of automatically recognizing and selecting a prosthesis target tooth from pre-preparation tooth data by using a margin line, according to an embodiment.
FIG. 8 is a reference diagram for explaining a method of obtaining an average normal based on a margin line, according to an embodiment.
FIG. 9 is a reference diagram for explaining a method of selecting a tooth area by using a seed point, according to an embodiment.
FIG. 10 is a flowchart illustrating the process of automatically recognizing and selecting a prosthesis target tooth from pre-preparation tooth data by using a margin line, according to an embodiment.
FIG. 11 is a reference diagram for explaining the process of generating a cylindrical three-dimensional figure surrounding a pre-preparation tooth, according to an embodiment.
FIG. 12 is a flowchart illustrating the process of automatically recognizing and selecting a prosthesis target tooth from a second intraoral image including preparation tooth data by using a margin line, according to an embodiment.
FIG. 13 is a reference diagram for explaining a method of obtaining an initial inner surface by cutting preparation tooth data by using a margin line, according to an embodiment.
FIG. 14 is a flowchart illustrating an example of a method of generating a prosthesis model by using a prosthesis target tooth automatically recognized from a first intraoral image and a second intraoral image, according to an embodiment.
FIG. 15 is a flowchart illustrating the process of obtaining the outer surface of a prosthesis model to restore a prosthesis target tooth by using surface data of the prosthesis target tooth automatically recognized from a first intraoral image based on a margin line, according to an embodiment.
FIG. 16 is a reference diagram for explaining a method of obtaining an initial outer surface from pre-preparation tooth data by using a margin line, according to an embodiment.
FIG. 17 is a reference diagram for explaining a method of generating a margin loop mesh by expanding a margin line, according to an embodiment.
FIG. 18 is a reference diagram for explaining a method of generating a cutting tool, according to an embodiment.
FIG. 19 is a reference diagram for explaining a method of obtaining a final outer surface by using a cutting tool, according to an embodiment.
FIG. 20 is a flowchart illustrating the process of obtaining surface data of a preparation tooth, which is the basis of the inner surface of a prosthesis model, from preparation tooth data by using a margin line, according to an embodiment.
FIG. 21 is a reference diagram for explaining a method of processing an initial inner surface, according to an embodiment.
FIG. 22 is a reference diagram for explaining an example of expanding a margin line, according to an embodiment.
FIG. 23 is a reference diagram for explaining a method of connecting the final inner surface of a prosthesis to the final outer surface of the prosthesis, according to an embodiment.

### BEST MODE

### MODE OF DISCLOSURE

This specification clarifies the scope of the inventive concept, explains the principles of the inventive concept, and discloses embodiments so that those of ordinary skill in the art to which the inventive concept pertains may practice the inventive concept. The disclosed embodiments may be implemented in various forms.

Like reference numerals refer to like elements throughout the specification. This specification does not describe all elements of the embodiments, and general content in the technical field to which the inventive concept pertains or content that overlaps among the embodiments is omitted. As used herein, the term "part" or "portion" may be implemented in software or hardware, and according to embodiments, a plurality of "units" may be implemented as one unit or element, or one "unit" may include a plurality of elements. Hereinafter, the working principle and embodiments of the inventive concept will be described with reference to the accompanying drawings.

In the present specification, the image may include at least one tooth or an image representing an oral cavity including at least one tooth (hereinafter, "intraoral image").

Also, in the present specification, an image may be a two-dimensional image of an object or a three-dimensional model or three-dimensional image representing the object three-dimensionally. Also, in the present specification, an image may refer to data necessary to represent an object in two or three dimensions, for example, raw data obtained from at least one image sensor. In particular, raw data is data obtained to generate an intraoral image and may be data (e.g., two-dimensional data) obtained from at least one image sensor included in an intraoral scanner when scanning the oral cavity of a patient, which is an object, using the intraoral scanner.

In the present specification, an "object" may include teeth, gingiva, at least a partial region of the oral cavity, and/or artificial structures (e.g., orthodontic appliances, implants, artificial teeth, orthodontic aids inserted into the mouth, etc.) that may be inserted into the oral cavity.

In the present specification, the 'intraoral image' may be composed of various polygonal meshes. For example, when two-dimensional data is obtained using an intraoral scanner, a data processing apparatus may calculate coordinates of a plurality of illuminated surface points using a triangulation method. As the amount of scan data increases by scanning the surface of the object while moving using the intraoral scanner, coordinates of the surface points may be accumulated. As a result of this image acquisition, a point cloud of vertices may be identified to represent the extent of the surface. Points in the point cloud may represent actual measured points on the three-dimensional surface of the object. The surface structure may be approximated by forming a polygonal mesh in which adjacent vertices of a point cloud are connected by line segments. The polygonal mesh may be variously determined, such as a triangular, quadrangular, or pentagonal mesh. The relationship between the polygons of the mesh model and the neighboring polygons may be used to extract features of the tooth boundary, for example, curvature, smallest curvature, edge, spatial relationship, and the like.

Hereinafter, embodiments will be described in detail with reference to the drawings.

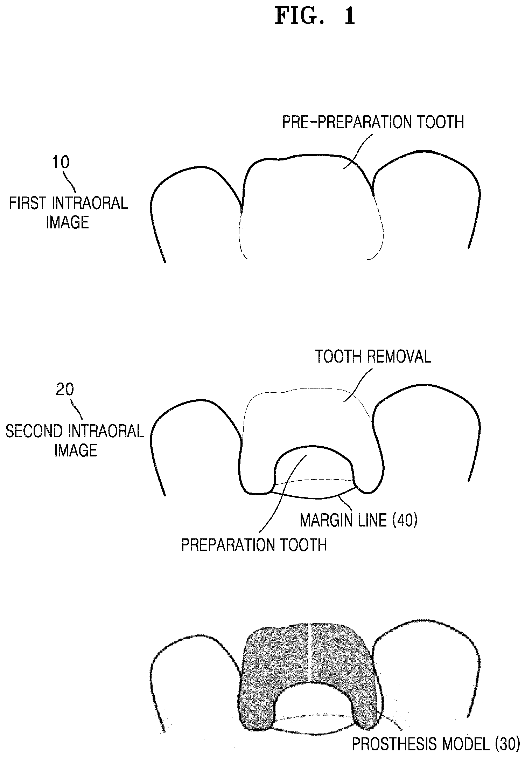

FIG. 1 is a reference diagram for explaining the creation of a prosthesis through tooth preparation, according to embodiments.

Tooth preparation refers to the process of generating space for planned restoration materials to restore the tooth to be restored to its original shape and function by cutting the tooth by removing the corrosion of the tooth or removing the structurally unstable part and may also be called "prep" for short.

A tooth before tooth preparation may be referred to as a pre-preparation tooth.

A tooth after tooth preparation operation may be referred to as a prepared tooth or a preparation tooth.

A dental prosthesis refers to a prosthesis that may artificially replace a tooth when the tooth is lost. For example, a crown refers to a tooth cap, a type of dental restoration that completely covers or surrounds a tooth or implant.

Referring to FIG. 1, a first intraoral image 10 represents an image obtained by scanning an oral cavity including a pre-preparation tooth, that is, a tooth before removal of a tooth to be restored.

A second intraoral image 20 represents an image obtained by scanning the oral cavity including a tooth prepared by removing the tooth to be restored. From the prepared tooth of the second intraoral image 20, a margin line indicating the boundary between a tooth and a prosthesis may be obtained. In general, the end of a removed part of the prepared tooth may be obtained as a margin line 40.

For example, a prosthesis such as a crown has a shape similar to the surface of a pre-preparation tooth and may be manufactured to surround a prepared tooth and meet the prepared tooth at the margin line 40. To design such a prosthesis, surface data of a tooth to be restored in the first intraoral image 10 is required. The surface data may be recorded in the form of a polygon mesh and may include location information on vertices of the surface of an object and connection relationship information on each vertex. Alternatively, the surface data may be recorded in the form of a point cloud and may include location information on vertices of the surface of the object.

In general, to obtain surface data of a tooth to be restored from the first intraoral image 10 obtained by scanning an oral cavity including a pre-preparation tooth, or to obtain surface data of a tooth to be restored from the second intraoral image 20 obtained by scanning an oral cavity including a preparation tooth, it may be necessary for a user to manually select each tooth to be restored. In particular, to make a prosthesis model, which is a restoration to replace a tooth to be restored, the tooth to be restored has to be specified from the first intraoral image 10 and the second intraoral image 20, and to specify the tooth to be restored, a selection process by the user may be necessary.

In the disclosed embodiments, a method is proposed to automatically recognize and select a prosthesis target tooth without requiring a separate selection operation by a user by automatically identifying the prosthesis target tooth from the first intraoral image 10 or the second intraoral image 20.

In the disclosed embodiments, a method of automatically recognizing and selecting a prosthesis target tooth from the first intraoral image 10 or the second intraoral image 20 is proposed.

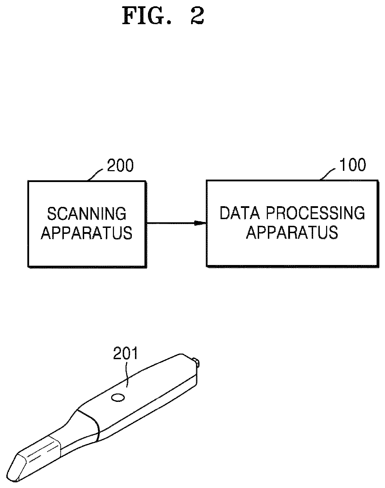

FIG. 2 is a diagram for explaining an intraoral image processing system according to an embodiment.

Referring to FIG. 2, the intraoral image processing system may include a scanning apparatus 200 and a data processing apparatus 100.

The scanning apparatus 200 scans an object, and the object may include any object or body to be scanned. For example, the object may include at least a part of a patient's body including an oral cavity or face, or a tooth model. The scanning apparatus 200 may include a handheld scanner for scanning an object while a user holds the handheld scanner, or a model scanner for installing a tooth model and scanning while moving around the installed tooth model.

For example, an intraoral scanner 201, which is a type of handheld scanner, may be a device for acquiring an image of an oral cavity including at least one tooth by being inserted into the oral cavity and scanning teeth in a non-contact manner. In addition, the intraoral scanner 201 may have a form that may be drawn in and out of the oral cavity, and scans the inside of the patient's oral cavity using at least one image sensor (e.g., an optical camera, etc.). The intraoral scanner 201 may acquire surface information on the object as raw data to image the surface of at least one of teeth, gingiva, and artificial structures (e.g., orthodontic devices including brackets and wires, implants, artificial teeth, orthodontic aids inserted into the oral cavity, etc.) insertable into the oral cavity, which are objects. The intraoral scanner 201 is suitable for scanning the oral cavity as it is in a form that is easy to enter and withdraw into the oral cavity. However, of course, body parts, such as the patient's face, may also be scanned using the intraoral scanner 201.

The scanning apparatus 200 may acquire image data using an optical triangulation method, a confocal method, or other methods.

The image data acquired by the scanning apparatus 200 may be transmitted to the data processing apparatus 100 connected through a wired or wireless communication network.

The data processing apparatus 100 may be any electronic device capable of being connected to the scanning apparatus 200 through a wired or wireless communication network, receiving a two-dimensional image obtained by scanning an oral cavity from the scanning apparatus 200, and generating, processing, displaying, and/or transmitting an intraoral image based on the received two-dimensional image.

The data processing apparatus 100 may generate at least one of information generated by processing two-dimensional image data and an intraoral image generated by processing two-dimensional image data based on the two-dimensional image data received from the scanning apparatus 200, and display the generated information and the intraoral image through a display.

The data processing apparatus 100 may be a computing device such as a smart phone, a laptop computer, a desktop computer, a PDA, or a tablet PC, but is not limited thereto.

Also, the data processing apparatus 100 may exist in the form of a server (or server apparatus) for processing an intraoral image.

Also, the scanning apparatus 200 may transmit raw data obtained through scanning to the data processing apparatus 100 as it is. In this case, the data processing apparatus 100 may generate a three-dimensional intraoral image representing the oral cavity three-dimensionally based on the received raw data. In addition, the "three-dimensional intraoral image" may be generated by modeling the internal structure of the oral cavity based on the received raw data in three dimensions, and may be referred to as a "three-dimensional intraoral model", a "digital intraoral model", or a "three-dimensional intraoral image". Hereinafter, a model or image representing the oral cavity in two or three dimensions is collectively referred to as an "intraoral image".

Also, the data processing apparatus 100 may analyze, process, display, and/or transmit the generated intraoral image to an external device.

As another example, the scanning apparatus 200 may acquire raw data through a scan, process the obtained raw data to generate an image corresponding to the oral cavity, which is an object, and transmit the image to the data processing apparatus 100. In this case, the data processing apparatus 100 may analyze, process, display, and/or transmit the received image.

In the disclosed embodiment, the data processing apparatus 100 is an electronic device capable of generating and displaying an intraoral image representing an oral cavity including one or more teeth in three dimensions, which will be described in detail below.

According to an embodiment, when the data processing apparatus 100 receives raw data obtained by scanning an oral cavity from the scanning apparatus 200, the data processing apparatus 100 may process the received raw data to generate an intraoral image representing a three-dimensional intraoral model. The raw data received from the scanning apparatus 200 may include raw data indicating teeth and raw data indicating gingiva. Accordingly, the intraoral image generated by the data processing apparatus 100 may include a tooth region representing a tooth and a gingiva region representing a gingiva.

According to an embodiment, the data processing apparatus 100 may automatically recognize and select a prosthesis target tooth from at least one of a first intraoral image including a pre-preparation tooth and a second intraoral image including a preparation tooth.

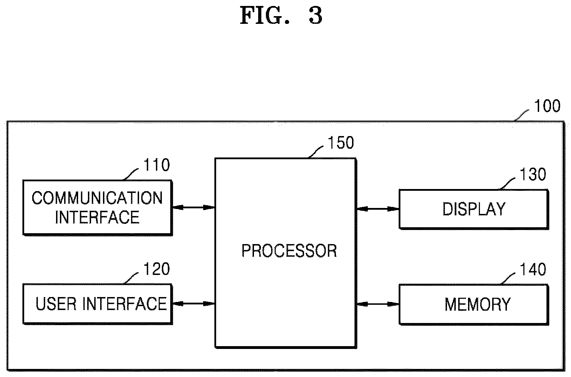

FIG. 3 is a block diagram illustrating a data processing apparatus 100 according to an embodiment.

Referring to FIG. 3, the data processing apparatus 100 may include a communication interface 110, a user interface 120, a display 130, a memory 140, and a processor 150.

The communication interface 110 may communicate with at least one external electronic device through a wired or wireless communication network. In particular, the communication interface 110 may communicate with the scanning apparatus 200 under the control of the processor 150. The communication interface 110 may communicate with an external electronic device or server connected through a wired/wireless communication network under the control of the processor.

The communication interface 110 may communicate with an external electronic device (e.g., an intraoral scanner, a server, or an external medical device) through a wired or wireless communication network. In particular, the communication interface may include at least one short-distance communication module for performing communication according to communication standards such as Bluetooth, Wi-Fi, Bluetooth Low Energy (BLE), NFC/RFID, Wi-Fi Direct, UWB, or ZIGBEE.

In addition, the communication interface 110 may further include a long-distance communication module that communicates with a server for supporting long-distance communication according to the long-distance communication standard. In particular, the communication interface 110 may include a long-distance communication module for performing communication through a network for Internet communication. In addition, the communication interface may include a long-distance communication module for performing communication through a communication network conforming to a communication standard such as 3G, 4G, and/or 5G.

In addition, the communication interface 110 may include at least one port for connecting to an external electronic device by a wired cable to communicate with an external electronic device (e.g., intraoral scanner, etc.) by wire. Accordingly, the communication interface 110 may communicate with an external electronic device connected by wire through at least one port.

The user interface 120 may receive a user input for controlling the data processing apparatus. The user interface 120 may include a user input device including a touch panel for sensing a user's touch, a button for receiving a user's push operation, a mouse or keyboard for designating or selecting a point on the user interface screen, and the like, but is not limited thereto.

Also, the user interface 120 may include a voice recognition device for voice recognition. For example, the voice recognition device may be a microphone, and the voice recognition device may receive a user's voice command or voice request. Accordingly, the processor may control an operation corresponding to a voice command or a voice request to be performed.

The display 130 displays a screen. In particular, the display 130 may display a preset screen under the control of the processor 150. In particular, the display 130 may display a user interface screen including an intraoral image generated based on data obtained by scanning the oral cavity of a patient in the scanning apparatus 200. Alternatively, the display 130 may display a user interface screen including information related to a patient's dental treatment.

The memory 140 may store at least one instruction. Also, the memory 140 may store at least one instruction to be executed by the processor. Also, the memory may store at least one program executed by the processor 150. In addition, the memory 140 may store data received from the intraoral scanner (e.g., raw data obtained through intraoral scan, etc.). Alternatively, the memory may store an intraoral image representing the oral cavity in three dimensions.

The processor 150 performs at least one instruction stored in the memory 140 to control an intended operation to be performed. Here, at least one instruction may be stored in an internal memory included in the processor 150 or a memory 140 included in the data processing apparatus separately from the processor.

In particular, the processor 150 may perform at least one instruction to control at least one configuration included in the data processing apparatus so that an intended operation is performed. Therefore, even if the processor performs preset operations as an example, the processor may control at least one component included in the data processing apparatus so that preset operations are performed.

According to an embodiment, the processor 150 may obtain a first intraoral image including pre-preparation tooth data and a second intraoral image including preparation tooth data by executing one or more instructions. According to an embodiment, the processor 150, by executing one or more instructions, may obtain a margin line based on the preparation tooth data. According to an embodiment, the processor 150, by executing one or more instructions, may automatically recognize and select a prosthesis target tooth from at least one of the first intraoral image and the second intraoral image based on the margin line.

According to an embodiment, the processor 150, by executing one or more instructions, may obtain the margin line by automatically recognizing the margin line based on the preparation tooth data or providing a user interface, which allows setting the margin line, and receiving a user input for setting the margin line through the user interface.

According to an embodiment, to automatically recognize and select the prosthesis target tooth from the first intraoral image based on the margin line, the processor 150, by executing one or more instructions, may obtain an intersection point where one or more normal vectors obtained based on the margin line intersect with the pre-preparation tooth data, and identify a tooth area of the prosthesis target tooth by using a curvature value of the obtained intersection point and a curvature value of points included in the pre-preparation tooth data.

According to an embodiment, to automatically recognize and select the prosthesis target tooth from the first intraoral image based on the margin line, the processor 150, by executing one or more instructions, may obtain a predefined figure surrounding the pre-preparation tooth data by using the margin line and automatically recognize the prosthesis target tooth by selecting the pre-preparation tooth data that falls within the predefined figure.

According to an embodiment, to automatically recognize and select the prosthesis target tooth from the second intraoral image based on the margin line, the processor 150, by executing one or more instructions, may select the prosthesis target tooth by cutting the preparation tooth data by using the margin line.

According to an embodiment, the processor 150, by executing one or more instructions, may generate an outer surface of a prosthesis model to restore the prosthesis target tooth by using the prosthesis target tooth automatically recognized from the first intraoral image based on the margin line, generate an inner surface of the prosthesis model by using the prosthesis target tooth automatically recognized from the second intraoral image based on the margin line, and generate the prosthesis model by using the outer surface of the prosthesis model and the inner surface of the prosthesis model.

According to an embodiment, to generate the outer surface of the prosthesis model, the processor 150, by executing one or more instructions, may obtain an initial outer surface from the pre-preparation tooth data by using the margin line, generate a margin loop mesh based on the margin line, generate a closed tooth by connecting the initial outer surface to the margin loop mesh, and generate the outer surface of the prosthesis model by removing a predefined area from the closed tooth.

According to an embodiment, to generate the inner surface of the prosthesis model, the processor 150, by executing one or more instructions, may obtain an initial inner surface by cutting the preparation tooth data by using the margin line, identify a cement area and a non-cement area in the initial inner surface, obtain an offset cement area by offsetting the cement area by a predetermined distance, and generate the inner surface of the prosthesis model by connecting the offset cement area to the non-cement area.

According to an example, the processor 150 may be implemented in a form that internally includes at least one internal processor and a memory device (e.g., RAM, ROM, etc.) for storing at least one of programs, instructions, signals, and data to be processed or used by the internal processor.

In addition, the processor 150 may include a graphic processing unit for processing a graphic corresponding to a video. In addition, the processor 150 may be implemented as a system on chip (SoC) in which a core and a GPU are integrated. Also, the processor 150 may include a single core or multiple cores. For example, the processor may include a dual-core, triple-core, quad-core, hexa-core, octa-core, deca-core, dodeca-core, hexa-dash-vale core, and the like.

In the disclosed embodiment, the processor 150 may generate an intraoral image based on a two-dimensional image received from the scanning apparatus 200.

In particular, under the control of the processor 150, the communication interface 110 may receive data obtained from the scanning apparatus 200, for example, raw data obtained through an intraoral scan. In addition, the processor 150 may generate a three-dimensional intraoral image representing the oral cavity three-dimensionally based on the raw data received from the communication interface. For example, to reconstruct a three-dimensional image according to the optical triangulation method, and the intraoral scanner may include at least one camera and, in a specific embodiment, may include an L camera corresponding to a left field of view and an R camera corresponding to a right field of view. In addition, the intraoral scanner may acquire L image data corresponding to the left field of view and R image data corresponding to the right field of view from the L camera and the R camera, respectively. Subsequently, the intraoral scanner (not shown) may transmit raw data including L image data and R image data to the communication interface of the data processing apparatus 100.

Then, the communication interface 110 may transmit the received raw data to the processor, and the processor may generate an intraoral image representing the oral cavity in three dimensions based on the received raw data.

In addition, the processor 150 may directly receive an intraoral image representing the oral cavity from an external server, a medical device, or the like by controlling the communication interface. In this case, the processor may acquire a three-dimensional intraoral image without generating a three-dimensional intraoral image based on the raw data.

According to the disclosed embodiment, that the processor 150 performs operations such as 'extract', 'acquire', and 'generate' may include controlling other components to perform the above-described operations in addition to directly performing the above-described operations by executing at least one instruction in the processor 150.

To implement the embodiments disclosed in the present disclosure, the data processing apparatus 100 may include only some of the components illustrated in FIG. 2, or may include more components in addition to the components illustrated in FIG. 2.

In addition, the data processing apparatus 100 may store and execute dedicated software linked to the intraoral scanner. Here, the dedicated software may be called a dedicated program, a dedicated tool, or a dedicated application. When the data processing apparatus 100 operates in conjunction with the scanning apparatus 200, dedicated software stored in the data processing apparatus 100 may be connected to the scanning apparatus 200 to receive data acquired through an intraoral scan in real time. For example, there is dedicated software for processing data acquired through intraoral scans in Medit's intraoral scanner. In particular, Medit produces and distributes dedicated software for processing, managing, using, and/or transmitting data acquired from intraoral scanners. Here, "dedicated software" refers to a program, tool, or application that may be operated in conjunction with the intraoral scanner, such that "dedicated software" may be commonly used by various intraoral scanners developed and sold by various manufacturers. In addition, the dedicated software described above may be produced and distributed separately from the intraoral scanner that performs the intraoral scan.

The data processing apparatus 100 may store and execute dedicated software corresponding to an intraoral scanner product. The dedicated software may perform at least one operation to acquire, process, store, and/or transmit the intraoral image. Here, the dedicated software may be stored in the processor. In addition, dedicated software may provide a user interface for use of data acquired from the intraoral scanner. Here, the user interface screen provided by the dedicated software may include an intraoral image generated according to the disclosed embodiment.

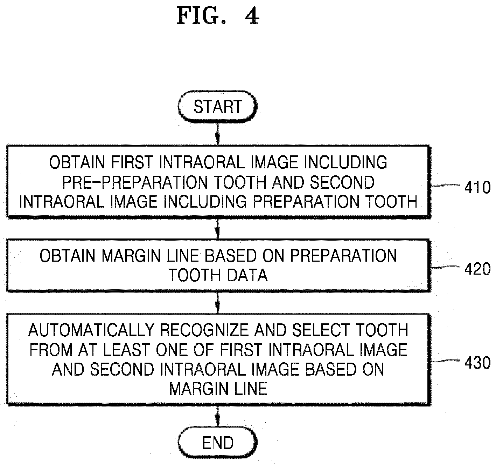

FIG. 4 is a flowchart illustrating a method of processing an intraoral image in a data processing apparatus according to an embodiment. The intraoral image processing method illustrated in FIG. 4 may be performed through the data processing apparatus 100. Accordingly, the intraoral image processing method illustrated in FIG. 4 may be a flowchart illustrating operations of the data processing apparatus 100.

Referring to FIG. 4, in operation 410, the data processing apparatus 100 may obtain a first intraoral image including a pre-preparation tooth and a second intraoral image including a preparation tooth.

The data processing apparatus 100 may receive, from the scanning apparatus 200, raw data obtained by scanning an intraoral in a patient or by scanning a tooth model and obtain an intraoral image by processing the received data. Alternatively, the data processing apparatus 100 may obtain an intraoral image stored in a memory.

According to an embodiment, the data processing apparatus 100 may acquire the first intraoral image obtained by scanning an oral cavity before preparing a tooth (hereinafter, referred to as a target tooth) to be restored. The first intraoral image may include a pre-preparation tooth. The data processing apparatus 100 may obtain the first intraoral image by processing in real time the raw data received from the scanning apparatus 200, or may obtain the first intraoral image by reading the first intraoral image pre-stored in the same form as library data from a memory.

According to an embodiment, the data processing apparatus 100 may acquire the second intraoral image obtained by scanning an oral cavity including a tooth (i.e., a preparation tooth) in which at least a portion of the target tooth has been removed by preparing the target tooth. The data processing apparatus 100 may obtain the second intraoral image by processing in real time the raw data received from the scanning apparatus 200, or may obtain the second intraoral image by reading the second intraoral image pre-stored in the same form as library data from a memory.

In operation 420, the data processing apparatus 100 may obtain a margin line based on preparation tooth data in the second intraoral image.

According to an embodiment, the data processing apparatus 100 may set the margin line automatically by a program or manually by user selection based on the preparation tooth data. For example, the data processing apparatus 100 may obtain a margin line by providing a user interface, which allows a user to set the margin line based on the preparation tooth data, and receiving a user input for setting the margin line through the user interface. For example, the data processing apparatus 100 may obtain a margin line by using an algorithm that extracts the margin line by calculating a curvature distribution by using surface information on the second intraoral image. In addition, the data processing apparatus 100 may edit the margin line by using one or more control points included in the margin line, regardless of whether the margin line is an automatically generated margin line or a manually generated margin line.

In operation 430, the data processing apparatus 100 may automatically recognize and select a prosthesis target tooth from at least one of the first intraoral image and the second intraoral image based on the margin line.

According to an embodiment, the data processing apparatus 100 may automatically recognize and select the prosthesis target tooth by obtaining surface data of the prosthesis target tooth from the first intraoral image including a pre-preparation tooth by using the margin line.

According to an embodiment, the data processing apparatus 100 may automatically recognize and select the prosthesis target tooth by obtaining surface data of the prosthesis target tooth from the second intraoral image including a preparation tooth by using the margin line.

According to the data processing apparatus 100 disclosed in this way, by identifying and recognizing the prosthesis target tooth based on the margin line without having to separately select a target tooth to be restored from the first intraoral image or the second intraoral image, the process of a user selecting a prosthesis target tooth may be eliminated, thereby increasing user convenience.

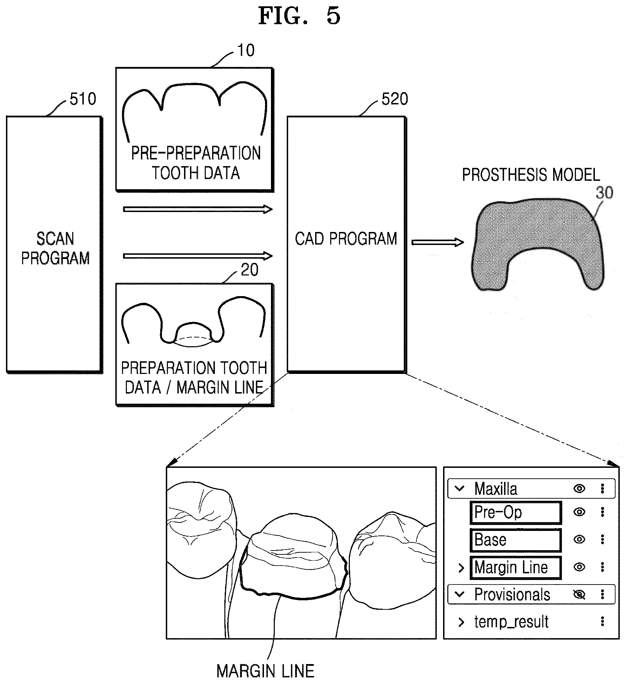

FIG. 5 illustrates an example of programs that generate a prosthesis model in the data processing apparatus 100, according to an embodiment.

Referring to FIG. 5, according to an embodiment, the data processing apparatus 100 may include a scan program 510 and a CAD program 520.

According to an embodiment, the scan program 510 may obtain a first intraoral image 10 by receiving, from the scanning apparatus 200, first oral cavity scan data obtained by scanning an oral cavity including a pre-preparation tooth and processing the received first oral cavity scan data.

According to an embodiment, the scan program 510 may obtain a second intraoral image 20 by receiving, from the scanning apparatus 200, second oral cavity scan data obtained by scanning an oral cavity including a preparation tooth and processing the received second oral cavity scan data.

According to an embodiment, the scan program 510 may automatically obtain a margin line from a preparation tooth included in the second intraoral image 20, or may manually obtain a margin line from the preparation tooth according to a user input.

According to an embodiment, the scan program 510 may output, to the CAD program 520, the first intraoral image 10, and the second intraoral image 20 in which a margin line is set.

According to an embodiment, the CAD program 520 may receive information on the first intraoral image 10, the second intraoral image 20, and the margin line from the scan program 510 and may automatically recognize and select a prosthesis target tooth based on the first intraoral image 10 and the second intraoral image 20 by using the margin line. Because the CAD program 520 may identify or process surface data of the prosthesis target tooth from the first intraoral image 10 and the second intraoral image 20 by using the margin line, the CAD program 520 may not require a separate target tooth selection process from a user. The CAD program 520 may generate a prosthesis model 30 to replace the prosthesis target tooth by using the first intraoral image 10, the second intraoral image 20, and the surface data of the recognized prosthesis target tooth.

In the example shown in FIG. 5, the scan program 510 obtains the margin line and transmits the margin line to the CAD program 520, but the embodiments are not limited thereto. The scan program 510 may transmit the first intraoral image 10 and the second intraoral image 20 to the CAD program 520 and the CAD program 520 may obtain a margin line.

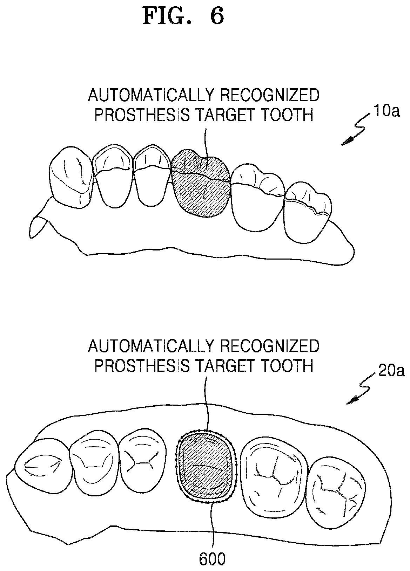

FIG. 6 is a reference diagram for explaining an example of automatically recognizing and displaying a prosthesis target tooth in a first intraoral image or a second intraoral image, according to an embodiment.

Referring to FIG. 6, the data processing apparatus 100 may automatically recognize a prosthesis target tooth in a first intraoral image 10a and provide a visual effect to the recognized prosthesis target tooth to indicate that the prosthesis target tooth has been selected.

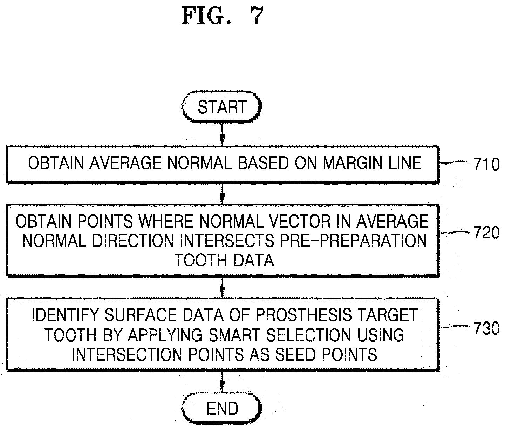

According to an embodiment, the data processing apparatus 100 may automatically recognize a prosthesis target tooth in the first intraoral image 10a based on a margin line. A method of automatically recognizing a prosthesis target tooth based on the margin line will be described in detail with reference to FIGS. 7 to 11.

According to an embodiment, the data processing apparatus 100 may display that a prosthesis target tooth has been selected from among the teeth in the first intraoral image 10a by adding a visual effect, such as color or border, to the prosthesis target tooth automatically recognized in the first intraoral image 10a.

The data processing apparatus 100 may automatically recognize a prosthesis target tooth in a second intraoral image 20a and provide a visual effect to the recognized prosthesis target tooth to indicate that the prosthesis target tooth has been selected.

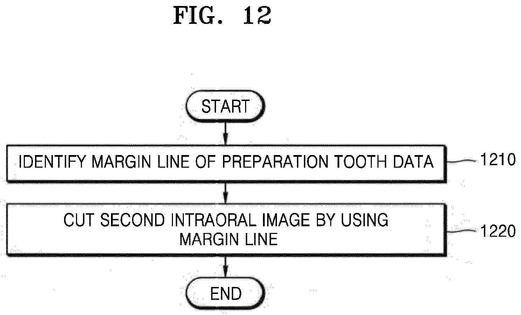

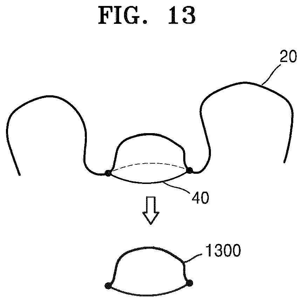

According to an embodiment, the data processing apparatus 100 may automatically recognize a prosthesis target tooth in the second intraoral image 20a based on a margin line. A method of automatically recognizing a prosthesis target tooth based on the margin line will be described in detail with reference to FIGS. 12 and 13.

According to an embodiment, the data processing apparatus 100 may display that a prosthesis target tooth has been selected from among the teeth in the second intraoral image 20a by adding a visual effect, such as color or border, to the prosthesis target tooth automatically recognized in the second intraoral image 20a. According to an embodiment, to display a prosthesis target tooth automatically recognized in the second intraoral image 20a, the data processing apparatus 100 may display a margin line 600 on the automatically recognized prosthesis target tooth. In addition, according to an embodiment, the data processing apparatus 100 may display the margin line 600 on a prosthesis target tooth automatically recognized in the second intraoral image 20a, and provide a user interface that allows the displayed margin line 600 to be modified according to user input.

Now, a method of automatically recognizing and selecting a prosthesis target tooth from pre-preparation tooth data by using a margin line will be described.

FIG. 7 is a flowchart illustrating the process of automatically recognizing and selecting a prosthesis target tooth from pre-preparation tooth data by using a margin line, according to an embodiment.

Referring to FIG. 7, in operation 710, the data processing apparatus 100 may obtain an average normal based on a margin line.

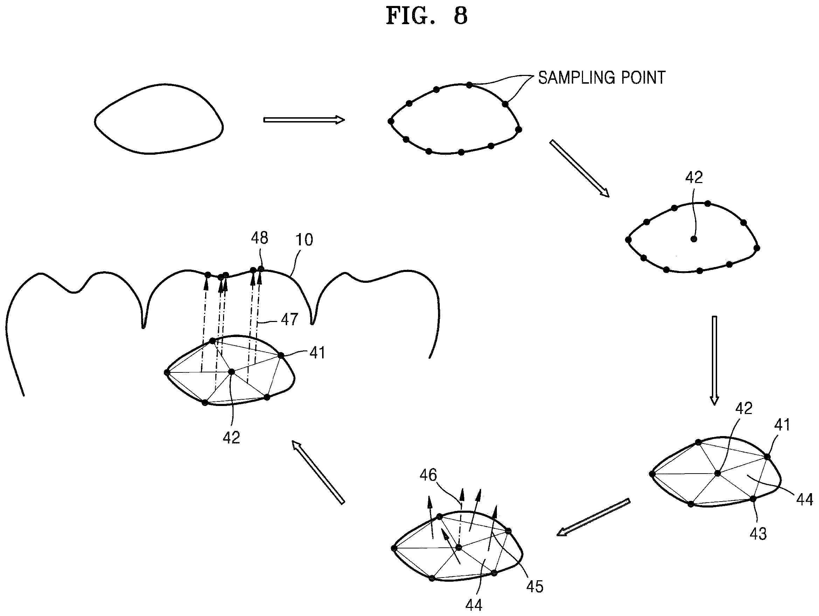

FIG. 8 is a reference diagram for explaining a method of obtaining an average normal based on a margin line, according to an embodiment.

Referring to FIG. 8, the data processing apparatus 100 may sample points (hereinafter, referred to as sampling points) constituting a margin line 40. In addition, the data processing apparatus 100 may calculate a loop center point 42 of the margin line 40 based on the coordinate values of the sampling points.

Next, the data processing apparatus 100 may select at least some of the sampling points and generate a mesh connecting the selected sampling points to the loop center point 42. Referring to FIG. 8, the data processing apparatus 100 may generate five meshes by selecting five sampling points and connecting each sampling point to the loop center point 42, according to an example. For example, the data processing apparatus 100 may generate a mesh 44 by connecting a sampling point 41, a sampling point 43, and a loop center point 42. The number of sampling points used to generate a mesh may or may not correspond to the number of seed points, which will be described later. The more seed points there are, the more accurate the results of smart selection may be, but when there are too many seed points, the amount of calculation increases, and thus, it may be important to select an appropriate number of seed points.

Next, the data processing apparatus 100 may calculate the average normal of each mesh. That is, the data processing apparatus 100 may obtain an average normal 46 by obtaining five normals corresponding to the five meshes and averaging the five normals. For example, the data processing apparatus 100 may obtain a normal 45 corresponding to the mesh 44. In the same way, the corresponding normals may be obtained for the remaining four meshes, respectively. Obtaining the average normal in this way relates to one example, and according to an embodiment, the data processing apparatus 100 may obtain the area normal of a curve (boundary line) forming a margin line.

Referring back to FIG. 7, in operation 720, the data processing apparatus 100 may obtain points where a normal vector in an average normal direction intersects pre-preparation tooth data.

Referring to FIG. 8, the data processing apparatus 100 may calculate points where pre-preparation tooth data intersects a normal vector heading in the average normal direction from the edge center between a sampling point and a loop center point. For example, the data processing apparatus 100 may calculate a point 48 where a normal vector 47 heading in the average normal direction from the edge center between the sampling point 41 and the loop center point 42 intersects a first intraoral image 10 including pre-preparation tooth data. By calculating intersection points for other sampling points in this manner, the data processing apparatus 100 may obtain five intersection points corresponding to the five selected sampling points.

Referring back to FIG. 7, in operation 730, the data processing apparatus 100 may identify surface data of a prosthesis target tooth by applying smart selection using intersection points as seed points. Smart selection refers to identifying a tooth area based on the curvature value of points that constitute the surface data of an intraoral image. Specifically, the data processing apparatus 100 may select a tooth area by determining a reference point based on a seed point and expanding a selection area based on the curvature value of the reference point. The tooth area may be the surface data of the prosthesis target tooth.

The data processing apparatus 100 according to an embodiment may determine a reference point on the intraoral image based on the curvature value of the seed point. Curvature is an indicator that indicates the degree to which a curved surface is curved and may be expressed as the reciprocal of the radius of the curved surface. The curvature value of a certain point on the surface of an object may represent the degree of bending of at least one curve that passes through the certain point and is determined on the surface of the object. In this case, the degree of bending of curves passing through the certain point may vary depending on the direction. Therefore, for example, the data processing apparatus 100 may determine the largest curvature value as the curvature value of the certain point, but is not limited thereto.

According to an embodiment, the data processing apparatus 100 may determine the seed point as the reference point when the curvature value of the seed point determined on an intraoral image is within a predefined range. On the other hand, when the curvature value of the seed point does not fall within the predefined range, the data processing apparatus 100 may determine another point having a curvature value within the predefined range as the reference point.

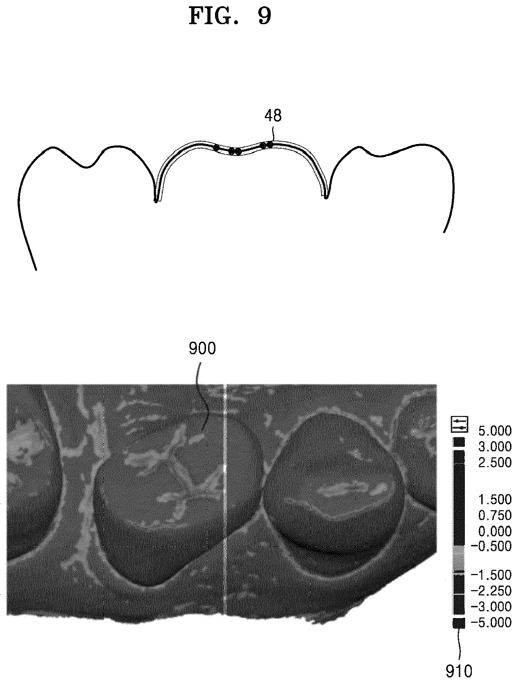

The data processing apparatus 100 may determine an area corresponding to an object, that is, a tooth, by gradually expanding a selection area having a curvature value within a critical range based on the curvature value of the reference point. Specifically, the data processing apparatus 100 may start from the reference point and expand the selection area by selecting adjacent points whose curvature values have a difference within a critical range from the curvature value of the reference point. The data processing apparatus 100 may no longer expand the selection area because the curvature value difference from the reference point is greater than the critical range when the selection area expands and reaches a tooth boundary area. Accordingly, the data processing apparatus 100 may identify a boundary area between teeth and gingiva. Referring to FIG. 9, the data processing apparatus 100 may display curvature values within a critical range in a predefined color (e.g., green) on a color bar 910 based on the curvature value of the reference point. In addition, the data processing apparatus 100 may display, in the same predefined color, an area 900 having curvature values within the critical range.

According to the method shown in FIG. 7, the surface area of the prosthesis target tooth corresponding to the margin line may be obtained by using curvature using some points of the intraoral image found based on the margin line of a tooth as seed points. In addition, it may be difficult to expand an area from a single seed point for a damaged tooth or a shape having severe valleys between teeth, and thus, the area may be expanded from seed points of various areas of a tooth by using a plurality of seed points and the entire tooth area may be selected by combining expanded areas.

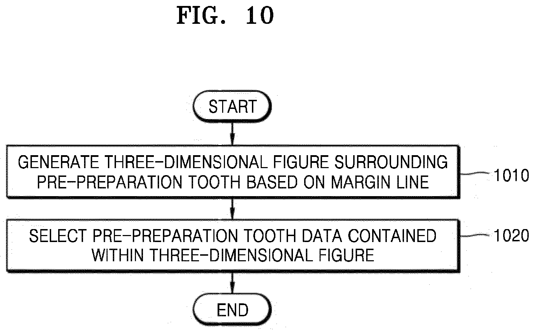

FIG. 10 is a flowchart illustrating the process of automatically recognizing and selecting a prosthesis target tooth from pre-preparation tooth data by using a margin line, according to an embodiment.

Referring to FIG. 10, in operation 1010, the data processing apparatus 100 may generate a cylindrical three-dimensional figure surrounding a pre-preparation tooth by using a margin line.

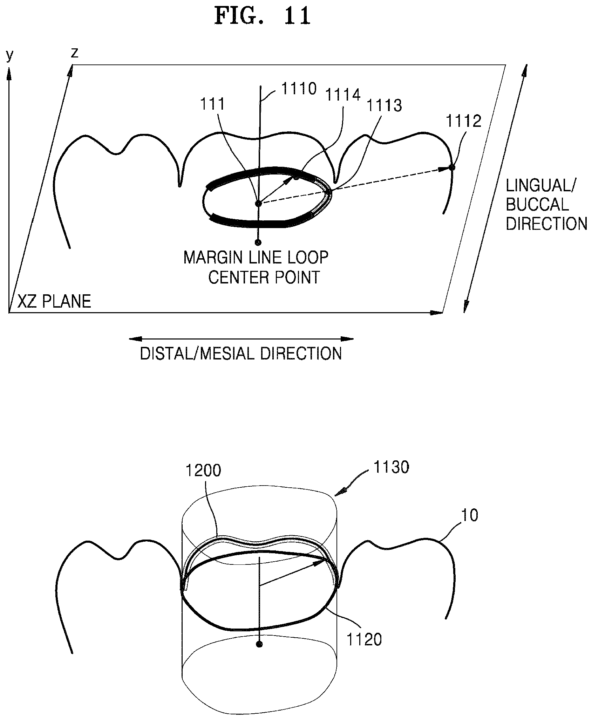

FIG. 11 is a reference diagram for explaining the process of generating a cylindrical three-dimensional figure surrounding a pre-preparation tooth, according to an embodiment.

Referring to FIG. 11, the data processing apparatus 100 may generate a loop having a predefined area based on points where a ray rotating 360 degrees on the x-z plane and pre-preparation tooth data intersect each other at each point on the y-axis 1110 passing through the center point of a margin line loop.

Because tooth surfaces in a buccal and lingual direction are scanned in the pre-preparation tooth data, the length where the ray meets the pre-preparation tooth data is relatively short (solid ray), and because tooth surfaces in a distal and mesial direction are not scanned due to adjacent teeth, the length where the ray meets the pre-preparation tooth data is relatively long (dotted ray). Therefore, when an intersection length exceeds a threshold value, a random point may be generated at an appropriate location. The data processing apparatus 100 may generate a loop based on randomly generated points and actual intersection points. For example, when a ray fired from a point 1111 on the y-axis 1110 in an occlusion direction is in the distal/mesial direction, the distance of the ray may exceed a threshold value by a point 1112 where the ray meets pre-preparation tooth data, and in this case, the data processing apparatus 100 may generate a point 1113 in an appropriate location. In addition, when the ray fired from the point 1111 on the y-axis 1110 is in the lingual/buccal direction, the point where the ray meets the pre-preparation tooth data may be a point 1114.

The data processing apparatus 100 may generate a loop corresponding to each point by performing the above process on all points on the y-axis in the occlusion direction.

The data processing apparatus 100 may select a loop 1120 having the largest area from among loops generated corresponding to each point on the y-axis 1110 and extend the loop 1120 in the y-axis direction to generate a three-dimensional figure 1130 having a predefined volume. According to an example, the three-dimensional figure 1130 may be a cylinder-shaped figure.

Referring back to FIG. 10, in operation 1020, the data processing apparatus 100 may automatically recognize surface data of a prosthesis target tooth by selecting pre-preparation tooth data that falls within a cylindrical three-dimensional figure.

Specifically, the data processing apparatus 100 may identify surface data 1200 of the prosthesis target tooth, which falls within a cylindrical three-dimensional figure 1130, from the first intraoral image 10 including pre-preparation tooth data, and the identified surface data 1200 of the prosthesis target tooth may be used as the external surface data of a prosthesis model.

FIG. 12 is a flowchart illustrating the process of automatically recognizing and selecting a prosthesis target tooth from a second intraoral image including preparation tooth data by using a margin line, according to an embodiment.

Referring to FIG. 12, in operation 1210, the data processing apparatus 100 may identify a margin line of preparation tooth data in the second intraoral image 20.

In operation 1220, the data processing apparatus 100 may automatically recognize and select a prosthesis target tooth by cutting the second intraoral image 20 using the margin line 40 to identify the surface data of the prosthesis target tooth. Specifically, the data processing apparatus 100 may recognize surface data 1300 (see FIG. 13) of a prosthesis target tooth by projecting a margin line 40 onto the preparation tooth data and cutting the preparation tooth data along a projected mesh. FIG. 13 illustrates the surface data 1300 of the prosthesis target tooth found by the process illustrated in FIG. 12.

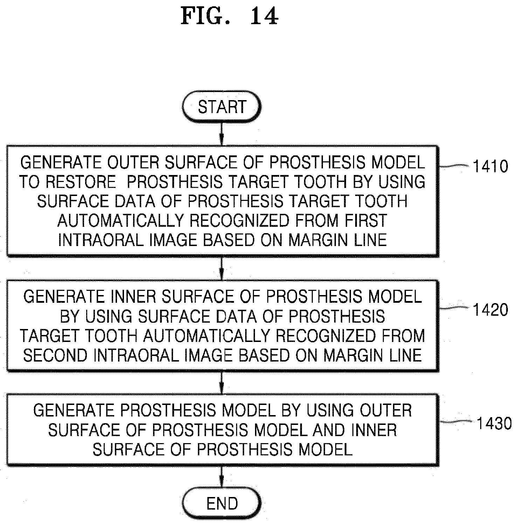

FIG. 14 is a flowchart illustrating an example of a method of generating a prosthesis model by using a prosthesis target tooth automatically recognized from a first intraoral image and a second intraoral image, according to an embodiment.

Referring to FIG. 14, in operation 1410, the data processing apparatus 100 may generate the outer surface of a prosthesis model to restore the prosthesis target tooth by using surface data of the prosthesis target tooth automatically recognized from the first intraoral image based on a margin line.

In operation 1420, the data processing apparatus 100 may generate the inner surface of the prosthesis model by using the surface data of the prosthesis target tooth automatically recognized from the second intraoral image based on the margin line.

In operation 1430, the data processing apparatus 100 may generate the prosthesis model by using the outer surface of the prosthesis model and the inner surface of the prosthesis model. The data processing apparatus 100 may generate the prosthesis model by connecting the outer surface of the prosthesis model to the inner surface of the prosthesis model.

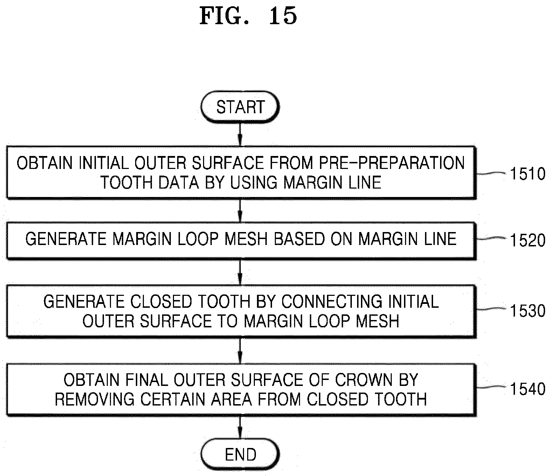

FIG. 15 is a flowchart illustrating the process of obtaining the outer surface of a prosthesis model to restore a prosthesis target tooth by using surface data of the prosthesis target tooth automatically recognized from a first intraoral image based on a margin line, according to an embodiment.

Referring to FIG. 15, in operation 1510, the data processing apparatus 100 may obtain the initial outer surface of the prosthesis model from the first intraoral image by using the margin line.

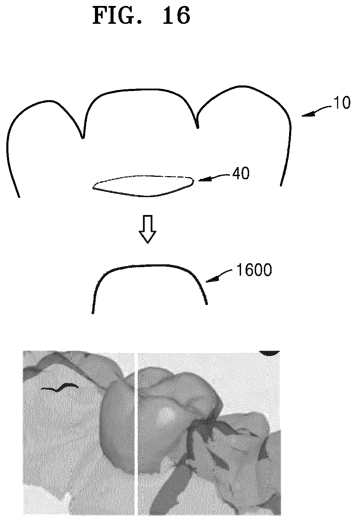

Referring to FIG. 16, the data processing apparatus 100 may obtain initial outer surface data 1600 of the prosthesis model by processing pre-preparation tooth data of a first intraoral image 10 by using a margin line 40. The method of obtaining the initial outer surface of the prosthesis model by processing pre-preparation tooth data by using the margin line 40 is the same as described with reference to FIGS. 7 to 11.

In operation 1520, the data processing apparatus 100 may generate a margin loop mesh by expanding the margin line 40.

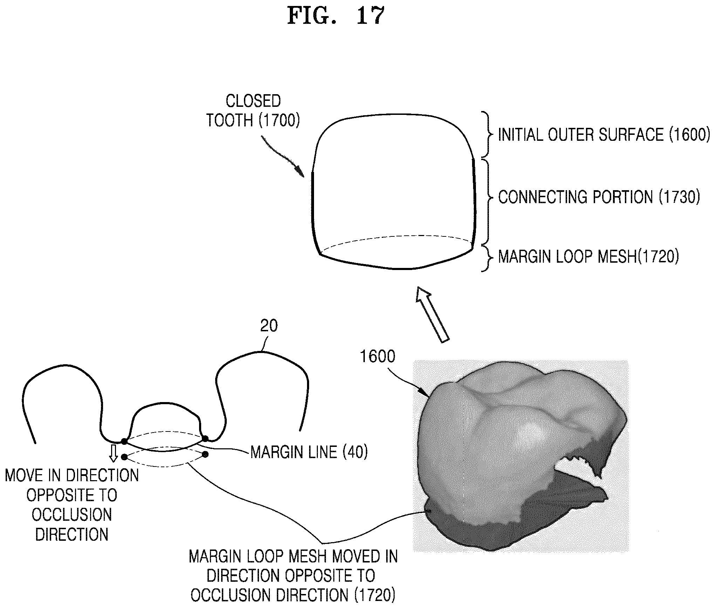

FIG. 17 is a reference diagram for explaining a method of generating a margin loop mesh by expanding a margin line, according to an embodiment.

Referring to FIG. 17, the data processing apparatus 100 may generate a margin loop mesh 1720 based on a margin line 40. According to an embodiment, the data processing apparatus 100 may obtain the margin loop mesh 1720, which is positioned lower than an original margin line, by moving the margin line 40 in a direction opposite to an occlusion direction.

In operation 1530, the data processing apparatus 100 may generate a closed tooth by connecting an initial outer surface to the margin loop mesh 1720 moved in the direction opposite to the occlusion direction.

Referring to FIG. 17, the data processing apparatus 100 may generate a closed tooth 1700, in which an interdental area is filled, by connecting, by using a connecting portion 1730, an initial outer surface 1600 of a crown to the margin loop mesh 1720 moved in the direction opposite to the occlusion direction.

In operation 1540, the data processing apparatus 100 may obtain the final outer surface of the crown by removing a predefined area from the closed tooth 1700.

Next, the data processing apparatus 100 may generate a cutting tool used to remove the predefined area from the closed tooth 1700.

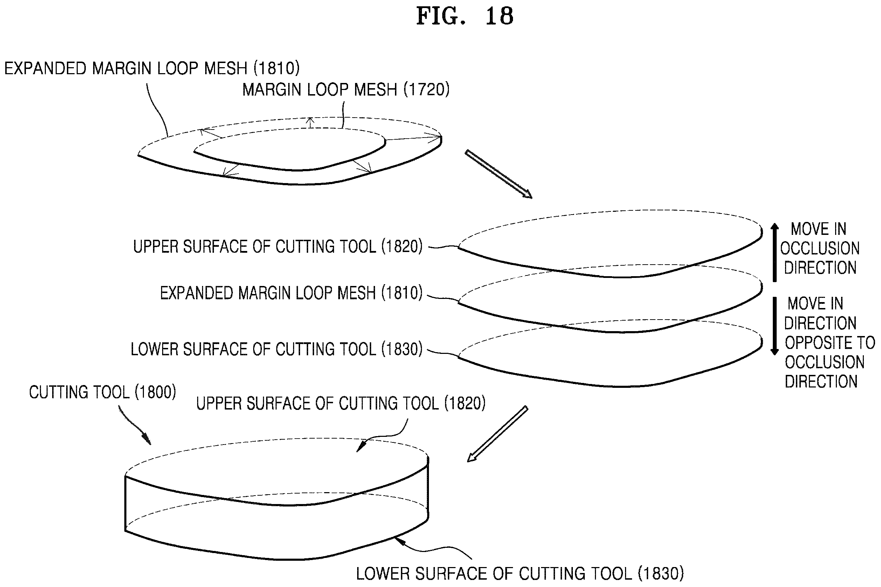

FIG. 18 is a reference diagram for explaining a method of generating a cutting tool, according to an embodiment.

Referring to FIG. 18, the data processing apparatus 100 may generate an expanded margin loop mesh 1810 by extending a margin loop mesh 1720 by a predefined distance in a circumferential direction.

Next, the data processing apparatus 100 may generate an upper surface 1820 of the cutting tool by moving the expanded margin loop mesh 1810 in the occlusion direction and may generate a lower surface 1830 of the cutting tool by moving the expanded margin loop mesh 1810 in a direction opposite to the occlusion direction.

Then, the data processing apparatus 100 may complete a cutting tool 1800 by connecting the upper surface 1820 of the cutting tool to the lower surface 1830 of the cutting tool. With this configuration, the upper surface of a cutting tool may be positioned higher than the height of a margin line. The reason why the upper surface of the cutting tool is positioned higher than the margin line is to make a more natural connection later when connecting the final outer surface of a prosthesis to the final inner surface of the prosthesis. When the height of the upper surface of the cutting tool is the same as or lower than the height of the margin line, a part connecting the final outer surface of the prosthesis to the final inner surface of the prosthesis may be sharply and unnaturally formed.

Next, the data processing apparatus 100 may obtain the final outer surface of the prosthesis by using the closed tooth and the cutting tool. Specifically, the data processing apparatus 100 may obtain the final outer surface by subjecting the closed tooth and the cutting tool 1800 to Boolean subtraction. Boolean is an operation that combines and models two objects, and Boolean subtraction may represent an operation that generates a surface by cutting off another object from one object.

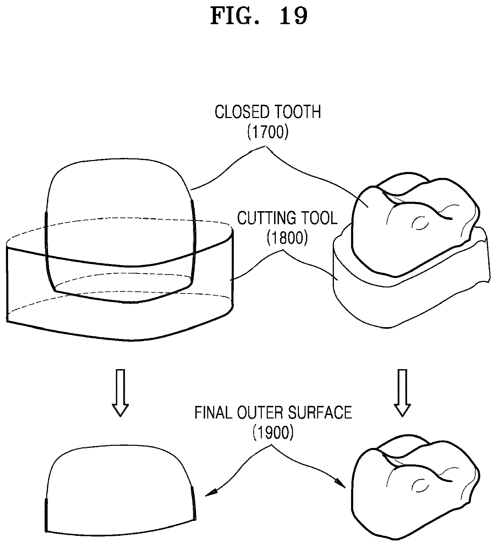

Referring to FIG. 19, the data processing apparatus 100 may obtain a final outer surface 1900 of a crown by performing Boolean subtraction to cut a cutting tool 1800 from a closed tooth 1700.

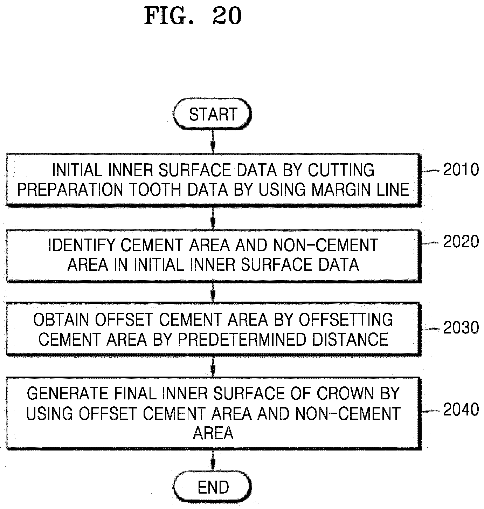

FIG. 20 is a flowchart illustrating the process of obtaining surface data of a preparation tooth, which is the basis of the inner surface of a prosthesis model, from preparation tooth data by using a margin line, according to an embodiment.

Referring to FIG. 20, in operation 2010, the data processing apparatus 100 may obtain initial inner surface data 800 of the prosthesis model by cutting the preparation tooth data in the second intraoral image 20 by using the margin line 40. Specifically, the data processing apparatus 100 may find initial inner surface data 1300 (see FIG. 13) by projecting the margin line 40 onto preparation tooth data and cutting the preparation tooth data along a projected mesh. FIG. 13 illustrates the initial inner surface data 1300 found by this method.

In operation 2020, the data processing apparatus 100 may identify a cement area and a non-cement area in the initial inner surface data.

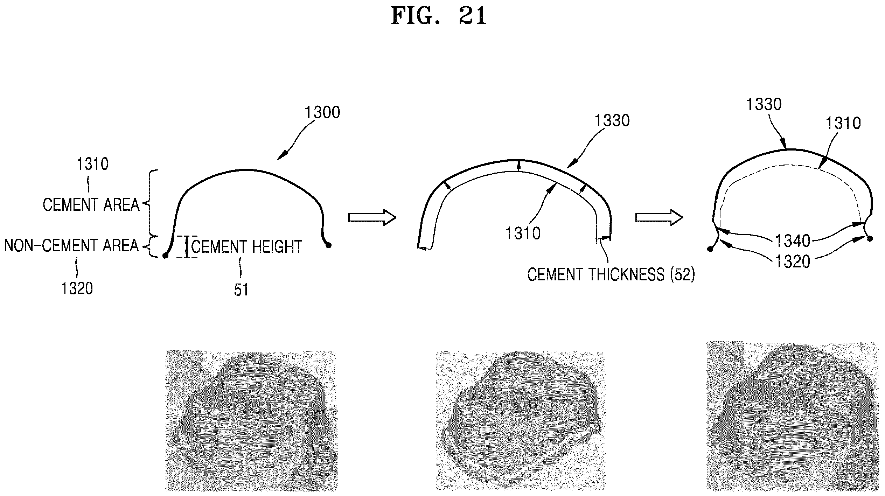

The cement area represents a section where there is a gap filled with cement to bond a crown to the preparation tooth. The data processing apparatus 100 may determine, as a cement height 51, a predefined distance from the boundary, that is, the margin line 40, in the initial inner surface data 1300 and may distinguish between the cement area and the non-cement area based on the cement height 51. Referring to FIG. 21, the data processing apparatus 100 may identify a cement area 1310 and a non-cement area 1320 in the initial inner surface data 1300.

In operation 2030, the data processing apparatus 100 may obtain an offset cement area by offsetting the cement area by a predetermined distance.

Referring to FIG. 21, the data processing apparatus 100 may obtain an offset cement area 1330 by offsetting the cement area 1310 by a cement thickness 52 to provide a space for filling the cement area 1310 with cement.

In operation 2040, the final inner surface of the crown may be generated using the offset cement area and the non-cement area.

Referring to FIG. 21, the data processing apparatus 100 may connect the offset cement area 1330 to the non-cement area 1320, and thus, the initial inner surface data of the prosthesis model may be composed of the non-cement area 1320, a connection portion 1340, and the cement area 1330.

Next, the data processing apparatus 100 may generate final inner surface data of the prosthesis model by extending the margin line from the initial inner surface data configured as described above. Generally, the crown is manufactured by milling, but when the crown is designed too tightly without free space, a milling tool may cause the crown to become smaller than an actual margin line, causing the problem of having to manufacture the crown again. Therefore, it is desirable to expand the margin line in the initial inner surface data of the prosthesis model to provide more margin.

According to an embodiment, the data processing apparatus 100 may expand the margin line by using a margin width and a margin angle.

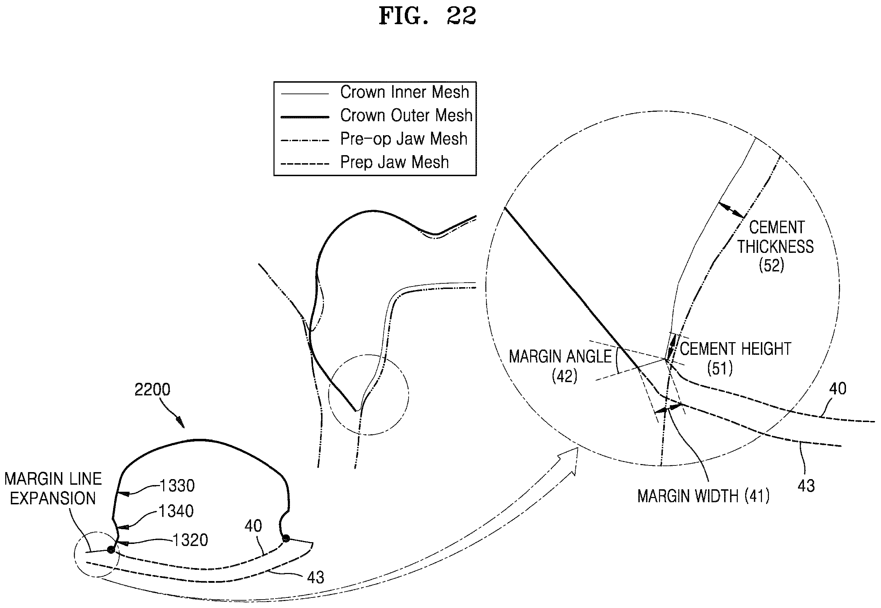

FIG. 22 is a reference diagram for explaining an example of expanding a margin line, according to an embodiment.

Referring to FIG. 22, the data processing apparatus 100 may extend a margin line 40 downward by a margin angle 42 and by a margin width 41. The size of the margin width 41 and the size of the margin angle 42 may be appropriately determined. For example, the margin angle 42 may be determined to be approximately 15 degrees. The data processing apparatus 100 may obtain an expanded margin line 43 by expanding the margin line 40 by a predetermined angle and a predetermined width. The data processing apparatus 100 may generate final inner surface data 1000 of the prosthesis by expanding the margin line in the initial inner surface data configured as described above.

According to an embodiment, the data processing apparatus 100 may determine appropriate values of the margin width 41, the margin angle 42, the cement height 51, and the cement thickness 52.

According to an embodiment, at least one of the margin width 41, the margin angle 42, the cement height 51, and the cement thickness 52 may be adjusted according to user input. Specifically, the data processing apparatus 100 may provide a user interface that allows adjustment of at least one of the margin width 41, the margin angle 42, the cement height 51, and the cement thickness 52, and may adjusts at least one of the margin width 41, the margin angle 42, the cement height 51, and the cement thickness 52 according to a user input received through the user interface.

Now, once the final inner surface of the prosthesis and the final outer surface of the prosthesis have been obtained, a prosthesis model may be generated by connecting the final inner surface of the prosthesis to the final outer surface of the prosthesis.

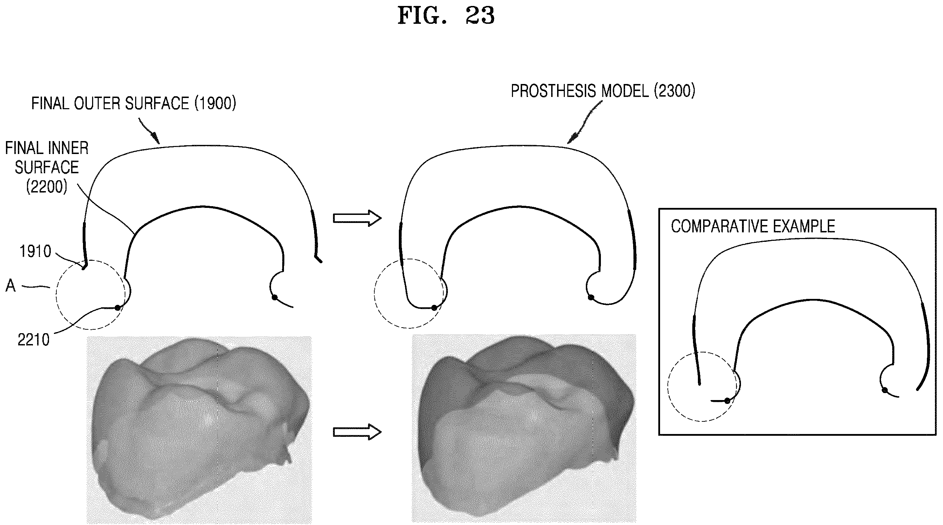

FIG. 23 is a reference diagram for explaining a method of connecting the final inner surface of the prosthesis to the final outer surface of the prosthesis, according to an embodiment.

Referring to FIG. 23, the data processing apparatus 100 may obtain a prosthesis model 2300 by connecting a final inner surface 2200 of a prosthesis model obtained as described with reference to FIG. 20 to the final outer surface 1900 of a crown obtained as described with reference to FIG. 15.

As previously described with reference to FIG. 15, the height of the upper surface of the cutting tool 1800 may be positioned higher than the margin line to generate the cutting tool 1800, and thus, the boundary of the final outer surface 1900 may be positioned higher than the margin line. By generating the final outer surface 1900 in this way, an end 1910 of the final outer surface 1900 is located at a higher position than an end 2210 of the final inner surface 2200. Therefore, when connecting the end 1910 of the final outer surface 1900 to the end 2210 of the final inner surface 2200, a smoother and more natural connection may be made.

As a comparative example, when the end 1910 of the final outer surface 1900 is at a position that is the same as or lower than the position of the margin line, the end 1910 of the final outer surface 1900 and the end 2210 of the final inner surface 2200 may be connected to each other more angularly, and thus, a connection portion may become unnatural. The inner surface data of a prosthesis generated using this method may be partially modified by considering an undercut area, a direction of insertion of the prosthesis, etc. In addition, when the thicknesses of the inner and outer surfaces of the prosthesis are less than a minimum thickness, some of the outer surface data may be expanded outward.

The intraoral image processing method according to an embodiment of the present disclosure may be implemented in the form of program instructions that may be executed through various computer means and recorded in a computer-readable medium. Also, an embodiment of the present disclosure may be a computer-readable storage medium in which one or more programs including at least one instruction for executing an intraoral image processing method are recorded.

The computer-readable storage medium may include program instructions, data files, data structures, etc. alone or in combination. Here, examples of computer-readable storage media may include hardware devices configured to store and execute program instructions, for example, magnetic media such as hard disks, floppy disks and magnetic tapes, optical media such as CD-ROM and DVD, magneto-optical media such as a floptical disk, and ROM, RAM, flash memory, etc.

Here, the device-readable storage medium may be provided in the form of a non-transitory storage medium. Here, the 'non-transitory storage medium' may mean that the storage medium is a tangible device. Also, the 'non-transitory storage medium' may include a buffer in which data is temporarily stored.

According to one embodiment, the intraoral image processing method according to various embodiments disclosed herein may be included in a computer program product and provided. The computer program product may be distributed in the form of a machine-readable storage medium (e.g., compact disc read only memory (CD-ROM)). Alternatively, the computer program produce may be distributed online (e.g., downloaded or uploaded) through an application store (e.g., play store, etc.) or directly between two user devices (e.g., smartphones). In particular, the computer program product according to the disclosed embodiment may include a storage medium in which a program including at least one instruction to perform the intraoral image processing method according to the disclosed embodiment is recorded.

Although embodiments have been described above in detail, the scope of the present disclosure is not limited thereto, and various modifications and alterations by those skill in the art using the basic concept of the present disclosure defined in the following claims also fall within the scope of the present disclosure.

## Claims

1. A method of processing an intraoral image (10,20), the method comprising:
obtaining a first intraoral image (10) including pre-preparation tooth data and a second intraoral image (20) including preparation tooth data;
obtaining a margin line (600) based on the preparation tooth data; and
automatically recognizing and selecting a prosthesis target tooth from at least one of the first intraoral image (10) and the second intraoral image (20) based on the margin line (600).

2. The method of claim 1, wherein the obtaining of the margin line based on the preparation tooth data comprises obtaining the margin line by automatically recognizing the margin line based on the preparation tooth data or providing a user interface, which allows setting the margin line, and receiving a user input for setting the margin line through the user interface.

3. The method of claim 1, wherein the automatically recognizing and selecting of the prosthesis target tooth from the first intraoral image based on the margin line comprises:
obtaining an intersection point where one or more normal vectors obtained based on the margin line intersect with the pre-preparation tooth data; and
identifying a tooth area of the prosthesis target tooth by using a curvature value of the obtained intersection point and a curvature value of points included in the pre-preparation tooth data.

4. The method of claim 1, wherein the automatically recognizing and selecting of the prosthesis target tooth from the first intraoral image based on the margin line comprises:
obtaining a predefined figure surrounding the pre-preparation tooth data by using the margin line; and
automatically recognizing the prosthesis target tooth by selecting the pre-preparation tooth data that falls within the predefined figure.

5. The method of claim 1, wherein the automatically recognizing and selecting of the prosthesis target tooth from the second intraoral image based on the margin line comprises selecting the prosthesis target tooth by cutting the preparation tooth data by using the margin line.

6. The method of claim 1, further comprising:
generating an outer surface of a prosthesis model to restore the prosthesis target tooth by using the prosthesis target tooth automatically recognized from the first intraoral image based on the margin line;
generating an inner surface of the prosthesis model by using the prosthesis target tooth automatically recognized from the second intraoral image based on the margin line; and
generating the prosthesis model by using the outer surface of the prosthesis model and the inner surface of the prosthesis model.

7. The method of claim 6, wherein the generating of the outer surface of the prosthesis model comprises;
obtaining an initial outer surface from the pre-preparation tooth data by using the margin line;
generating a margin loop mesh based on the margin line;
generating a closed tooth by connecting the initial outer surface to the margin loop mesh; and
generating the outer surface of the prosthesis model by removing a predefined area from the closed tooth.

8. The method of claim 6, wherein the generating of the inner surface of the prosthesis model comprises:
obtaining an initial inner surface by cutting the preparation tooth data by using the margin line;
identifying a cement area and a non-cement area in the initial inner surface;
obtaining an offset cement area by offsetting the cement area by a predetermined distance; and
generating the inner surface of the prosthesis model by connecting the offset cement area to the non-cement area.

9. A data processing apparatus for processing an intraoral image (10,20), the data processing apparatus comprising:
a memory including one or more instructions; and
a processor configured to, by executing the one or more instructions:
obtain a first intraoral image (10) including pre-preparation tooth data and a second intraoral image (20) including preparation tooth data:
obtain a margin line (600) based on the preparation tooth data; and
automatically recognize and select a prosthesis target tooth from at least one of the first intraoral image (10) and the second intraoral image (20) based on the margin line (600).

10. The data processing apparatus of claim 9, wherein the processor is further configured to, by executing the one or more instructions, obtain the margin line by automatically recognizing the margin line based on the preparation tooth data or providing a user interface, which allows setting the margin line, and receiving a user input for setting the margin line through the user interface.

11. The data processing apparatus of claim 9, wherein, to automatically recognize and select the prosthesis target tooth from the first intraoral image based on the margin line, the processor is further configured to, by executing the one or more instructions:
obtain an intersection point where one or more normal vectors obtained based on the margin line intersect with the pre-preparation tooth data; and
identify a tooth area of the prosthesis target tooth by using a curvature value of the obtained intersection point and a curvature value of points included in the pre-preparation tooth data.

12. The data processing apparatus of claim 9, wherein, to automatically recognize and select the prosthesis target tooth from the first intraoral image based on the margin line, the processor is further configured to, by executing the one or more instructions:
obtain a predefined figure surrounding the pre-preparation tooth data by using the margin line; and
automatically recognize the prosthesis target tooth by selecting the pre-preparation tooth data that falls within the predefined figure.

13. The data processing apparatus of claim 9, wherein, to automatically recognize and select the prosthesis target tooth from the second intraoral image based on the margin line, the processor is further configured to, by executing the one or more instructions, select the prosthesis target tooth by cutting the preparation tooth data by using the margin line.

14. The data processing apparatus of claim 9, wherein the processor is further configured to, by executing the one or more instructions:
generate an outer surface of a prosthesis model to restore the prosthesis target tooth by using the prosthesis target tooth automatically recognized from the first intraoral image based on the margin line;
generate an inner surface of the prosthesis model by using the prosthesis target tooth automatically recognized from the second intraoral image based on the margin line; and
generate the prosthesis model by using the outer surface of the prosthesis model and the inner surface of the prosthesis model.

15. A non-transitory computer-readable recording medium having stored thereon a program which, when executed, causes a computing apparatus to perform operations, the operations comprising:
obtaining a first intraoral image (10) including pre-preparation tooth data and a second intraoral image (20) including preparation tooth data;
obtaining a margin line (600) based on the preparation tooth data; and
automatically recognizing and selecting a prosthesis target tooth from at least one of the first intraoral image (10) and the second intraoral image (20) based on the margin line (600).

## Patentansprüche

1. Verfahren zum Verarbeiten eines intraoralen Bildes (10,20), wobei das Verfahren umfasst:
Erhalten eines ersten intraoralen Bildes (10) einschließlich Vorpräparationszahndaten und eines zweiten intraoralen Bildes (20) einschließlich Präparationszahndaten;
Erhalten einer Randlinie (600)
auf der Grundlage der Präparationszahndaten; und
automatisches Erkennen und Auswählen eines Prothesenzielzahns aus mindestens einem von dem ersten intraoralen Bild (10) und dem zweiten intraoralen Bild (20) auf der Grundlage der Randlinie (600).

2. Verfahren nach Anspruch 1, wobei das Erhalten der Randlinie auf der Grundlage der Präparationszahndaten das Erhalten der Randlinie durch automatisches Erkennen der Randlinie auf der Grundlage der Präparationszahndaten oder das Bereitstellen einer Benutzerschnittstelle, die das Einstellen der Randlinie ermöglicht, und das Empfangen einer Benutzereingabe zum Einstellen der Randlinie über die Benutzerschnittstelle umfasst.

3. Verfahren nach Anspruch 1, wobei das automatische Erkennen und Auswählen des Prothesenzielzahns aus dem ersten intraoralen Bild auf der Grundlage der Randlinie Folgendes umfasst:
Erhalten eines Schnittpunkts, an dem sich ein oder mehrere Normalvektoren, die auf Grundlage der Randlinie erhalten werden, mit den Vorpräparationszahndaten schneiden; und
Identifizieren eines Zahnbereichs des Prothesenzielzahns unter Verwendung eines Krümmungswertes des erhaltenen Schnittpunkts und eines Krümmungswertes von Punkten, die in den Präparationszahndaten enthalten sind.

4. Verfahren nach Anspruch 1, wobei das automatische Erkennen und Auswählen des Prothesenzielzahns aus dem ersten intraoralen Bild auf der Grundlage der Randlinie Folgendes umfasst:
Erhalten einer vordefinierten Figur, die die Vorpräparationszahndaten umgibt, unter Verwendung der Randlinie; und
automatisches Erkennen des Prothesenzielzahns, indem die Präparationszahndaten ausgewählt werden, die innerhalb der vordefinierten Figur liegen.

5. Verfahren nach Anspruch 1, wobei das automatische Erkennen und Auswählen des Prothesenzielzahns aus dem zweiten intraoralen Bild auf der Grundlage der Randlinie das Auswählen des Prothesenzielzahns durch Schneiden der Präparationszahndaten unter Verwendung der Randlinie umfasst.

6. Verfahren nach Anspruch 1, ferner umfassend:
Erzeugen einer Außenfläche eines Prothesenmodells, um den Prothesenzielzahn wiederherzustellen, unter Verwendung des Prothesenzielzahns, der automatisch aus dem ersten intraoralen Bild auf der Grundlage der Randlinie erkannt wurde;
Erzeugen einer Innenfläche des Prothesenmodells unter Verwendung des Prothesenzielzahns, der automatisch aus dem zweiten intraoralen Bild auf der Grundlage der Randlinie erkannt wurde; und
Erzeugen des Prothesenmodells unter Verwendung der Außenfläche des Prothesenmodells und der Innenfläche des Prothesenmodells.

7. Verfahren nach Anspruch 6, wobei das Erzeugen der Außenfläche des Prothesenmodells Folgendes umfasst;
Erhalten einer anfänglichen Außenfläche aus den Vorpräparationszahndaten unter Verwendung der Randlinie;
Erzeugen einer Präparationsrandschleife auf der Grundlage der Randlinie;
Erzeugen eines geschlossenen Zahns durch Verbinden der anfänglichen Außenfläche mit der Präparationsrandschleife; und
Erzeugen der Außenfläche des Prothesenmodells durch Entfernen eines vordefinierten Bereichs von dem geschlossenen Zahn.

8. Verfahren nach Anspruch 6, wobei das Erzeugen der Innenfläche des Prothesenmodells Folgendes umfasst:
Erhalten einer anfänglichen Innenfläche durch Schneiden der Präparationszahndaten unter Verwendung der Randlinie;
Identifizieren eines Zementbereichs und eines Nicht-Zementbereichs in der anfänglichen Innenfläche;
Erhalten eines versetzten Zementbereichs durch Versetzen des Zementbereichs um einen vorbestimmten Abstand; und
Erzeugen der Innenfläche des Prothesenmodells durch Verbinden des versetzten Zementbereichs mit dem Nicht-Zementbereich.

9. Datenverarbeitungsgerät zum Verarbeiten eines intraoralen Bildes (10,20), wobei das Datenverarbeitungsgerät umfasst:
einen Speicher, der eine oder mehrere Anweisungen beinhaltet; und
einen Prozessor, der so konfiguriert ist, dass er durch Ausführen der einen oder mehreren Anweisungen:
ein erstes intraorales Bild (10), das Vorpräparationszahndaten beinhaltet, und ein zweites intraorales Bild (20), das Präparationszahndaten beinhaltet, erhält;
eine Randlinie (600) auf der Grundlage der Präparationszahndaten erhält; und
einen Prothesenzielzahn aus mindestens einem des ersten intraoralen Bildes (10) und des zweiten intraoralen Bildes (20) auf der Grundlage der Randlinie (600) automatisch erkennt und auswählt.

10. Datenverarbeitungsgerät nach Anspruch 9, wobei der Prozessor ferner so konfiguriert ist, dass er durch Ausführen der einen oder mehreren Anweisungen die Randlinie durch automatisches Erkennen der Randlinie auf Grundlage der Präparationszahndaten oder Bereitstellen einer Benutzerschnittstelle, die das Einstellen der Randlinie ermöglicht, und Empfangen einer Benutzereingabe zum Einstellen der Randlinie über die Benutzerschnittstelle erhält.

11. Datenverarbeitungsgerät nach Anspruch 9, wobei zum automatischen Erkennen und Auswählen des Prothesenzielzahns aus dem ersten intraoralen Bild auf Grundlage der Randlinie der Prozessor ferner so konfiguriert ist, dass er durch Ausführen der einen oder mehreren Anweisungen:
einen Schnittpunkt erhält, an dem sich ein oder mehrere Normalvektoren, die auf Grundlage der Randlinie erhalten werden, mit den Vorpräparationszahndaten schneiden; und
einen Zahnbereich des Prothesenzielzahns unter Verwendung eines Krümmungswerts des erhaltenen Schnittpunkts und eines Krümmungswerts von Punkten, die in den Präparationszahndaten enthalten sind, identifiziert.

12. Datenverarbeitungsgerät nach Anspruch 9, wobei zum automatischen Erkennen und Auswählen des Prothesenzielzahns aus dem ersten intraoralen Bild auf Grundlage der Randlinie der Prozessor ferner so konfiguriert ist, dass er durch Ausführen der einen oder mehreren Anweisungen:
unter Verwendung der Randlinie eine vordefinierte Figur erhält, die die Vorpräparationszahndaten umgibt; und
automatisch den Prothesenzielzahn erkennt, indem die Präparationszahndaten ausgewählt werden, die innerhalb der vordefinierten Figur liegen.

13. Datenverarbeitungsgerät nach Anspruch 9, wobei der Prozessor zum automatischen Erkennen und Auswählen des Prothesenzielzahns aus dem zweiten intraoralen Bild auf Grundlage der Randlinie ferner so konfiguriert ist, dass er durch Ausführen der einen oder mehreren Anweisungen den Prothesenzielzahn durch Schneiden der Präparationszahndaten unter Verwendung der Randlinie auswählt.

14. Datenverarbeitungsgerät nach Anspruch 9, wobei der Prozessor ferner so konfiguriert ist, dass er durch Ausführen der einen oder mehreren Anweisungen:
eine Außenfläche eines Prothesenmodells erzeugt, um den Prothesenzielzahn wiederherzustellen, indem der Prothesenzielzahn verwendet wird, der automatisch aus dem ersten intraoralen Bild auf der Grundlage der Randlinie erkannt wurde;
eine Innenfläche des Prothesenmodells unter Verwendung des Prothesenzielzahns erzeugt, der automatisch aus dem zweiten intraoralen Bild auf der Grundlage der Randlinie erkannt wurde; und
das Prothesenmodell unter Verwendung der Außenfläche des Prothesenmodells und der Innenfläche des Prothesenmodells erzeugt.

15. Nicht-transitorisches computerlesbares Aufzeichnungsmedium, auf dem ein Programm gespeichert ist, das bei Ausführung eine Rechengerät veranlasst, Operationen durchzuführen, wobei die Operationen umfassen:
Erhalten eines ersten intraoralen Bildes (10)
einschließlich Vorpräparationszahndaten und eines
zweiten intraoralen Bildes (20) einschließlich Präparationszahndaten;
Erhalten einer Randlinie (600)
auf der Grundlage der Präparationszahndaten; und
automatisches Erkennen und Auswählen eines Prothesenzielzahns aus mindestens einem von dem ersten intraoralen Bild (10) und dem zweiten intraoralen Bild (20) auf der Grundlage der Randlinie (600).

## Revendications

1. Procédé de traitement d'une image intrabuccale (10,20), le procédé comprenant :
l'obtention d'une première image intrabuccale (10) comprenant des données de dent de pré-préparation et d'une seconde image intrabuccale (20) comprenant des données de dent de préparation :
l'obtention d'une ligne de marge (600)
sur la base des données de dent de préparation ; et
la reconnaissance et la sélection automatiques d'une dent cible prothétique à partir d'au moins l'une de la première image intrabuccale (10) et de la seconde image intrabuccale (20) sur la base de la ligne de marge (600).

2. Procédé de la revendication 1, ladite obtention de la ligne de marge sur la base des données de dent de préparation comprenant l'obtention de la ligne de marge en reconnaissant automatiquement la ligne de marge sur la base des données de dent de préparation ou en fournissant une interface utilisateur, qui permet de définir la ligne de marge, et en recevant une entrée utilisateur pour définir la ligne de marge par l'intermédiaire de l'interface utilisateur.

3. Procédé de la revendication 1, ladite reconnaissance et ladite sélection automatiques de la dent cible prothétique à partir de la première image intrabuccale sur la base de la ligne de marge comprenant :
l'obtention d'un point d'intersection où un ou plusieurs vecteurs normaux obtenus sur la base de la ligne de marge croisent les données de dent de pré-préparation ; et
l'identification d'une zone de dent de la dent cible prothétique à l'aide d'une valeur de courbure du point d'intersection obtenu et une valeur de courbure de points compris dans les données de dent de pré-préparation.

4. Procédé de la revendication 1, ladite reconnaissance et ladite sélection automatiques de la dent cible prothétique à partir de la première image intrabuccale sur la base de la ligne de marge comprenant :
l'obtention d'une figure prédéfinie entourant les données de dent de pré-préparation à l'aide de la ligne de marge ; et
la reconnaissance automatique de la dent cible prothétique en sélectionnant les données de dent de pré-préparation qui se rapportent à la figure prédéfinie.

5. Procédé de la revendication 1, ladite reconnaissance et ladite sélection automatiques de la dent cible prothétique à partir de la seconde image intrabuccale sur la base de la ligne de marge comprenant la sélection de la dent cible prothétique en coupant les données de dent de préparation à l'aide de la ligne de marge.

6. Procédé de la revendication 1, comprenant en outre :
la génération d'une surface externe d'un modèle de prothèse pour restaurer la dent cible prothétique à l'aide de la dent cible prothétique reconnue automatiquement à partir de la première image intrabuccale sur la base de la ligne de marge ;
la génération d'une surface interne du modèle de prothèse à l'aide de la dent cible prothétique reconnue automatiquement à partir de la seconde image intrabuccale sur la base de la ligne de marge ; et
la génération du modèle de prothèse à l'aide de la surface externe du modèle de prothèse et la surface interne du modèle de prothèse.

7. Procédé de la revendication 6, ladite génération de la surface externe du modèle de prothèse comprenant ;
l'obtention d'une surface externe initiale à partir des données de dent de pré-préparation à l'aide de la ligne de marge ;
la génération d'un maillage de boucle de marge sur la base de la ligne de marge ;
la génération d'une dent fermée en reliant la surface externe initiale au maillage de boucle de marge ; et
la génération de la surface externe du modèle de prothèse en supprimant une zone prédéfinie de la dent fermée.

8. Procédé de la revendication 6, ladite génération de la surface interne du modèle de prothèse comprenant :
l'obtention d'une surface interne initiale en coupant les données de dent de préparation à l'aide de la ligne de marge ;
l'identification d'une zone de ciment et d'une zone sans ciment dans la surface interne initiale ;
l'obtention d'une zone de ciment décalée en décalant la zone de ciment d'une distance prédéfinie ; et
la génération de la surface interne du modèle de prothèse en reliant la zone de ciment décalée à la zone sans ciment.

9. Appareil de traitement de données destiné au traitement d'une image intrabuccale (10,20), l'appareil de traitement de données comprenant :
une mémoire comprenant une ou plusieurs instructions ; et
un processeur configuré pour, en exécutant la ou les instructions :
obtenir une première image intrabuccale (10) comprenant des données de dent de pré-préparation et une seconde image intrabuccale (20) comprenant des données de dent de préparation ;
obtenir une ligne de marge (600)
sur la base des données de dent de préparation ; et
reconnaître et sélectionner automatiquement une dent cible prothétique à partir d'au moins l'une de la première image intrabuccale (10) et de la seconde image intrabuccale (20) sur la base de la ligne de marge (600).

10. Appareil de traitement de données de la revendication 9, ledit processeur étant en outre configuré pour, en exécutant la ou les instructions, obtenir la ligne de marge en reconnaissant automatiquement la ligne de marge sur la base des données de dent de préparation ou en fournissant une interface utilisateur, qui permet de définir la ligne de marge, et en recevant une entrée utilisateur pour définir la ligne de marge par l'intermédiaire de l'interface utilisateur.

11. Appareil de traitement de données de la revendication 9, pour reconnaître et sélectionner automatiquement la dent cible prothétique à partir de la première image intrabuccale sur la base de la ligne de marge, ledit processeur étant en outre configuré pour, en exécutant la ou les instructions :
obtenir un point d'intersection où un ou plusieurs vecteurs normaux obtenus sur la base de la ligne de marge croisent les données de dent de pré-préparation ; et
identifier une zone de dent de la dent cible prothétique à l'aide d'une valeur de courbure du point d'intersection obtenu et d'une valeur de courbure de points compris dans les données de dent de pré-préparation.

12. Appareil de traitement de données de la revendication 9, pour reconnaître et sélectionner automatiquement la dent cible prothétique à partir de la première image intrabuccale sur la base de la ligne de marge, ledit processeur étant en outre configuré pour, en exécutant la ou les instructions :
obtenir une figure prédéfinie entourant les données de dent de pré-préparation à l'aide de la ligne de marge ; et
reconnaître automatiquement la dent cible prothétique en sélectionnant les données de dent de pré-préparation qui se rapportent à la figure prédéfinie.

13. Appareil de traitement de données de la revendication 9, pour reconnaître et sélectionner automatiquement la dent cible prothétique à partir de la seconde image intrabuccale sur la base de la ligne de marge, ledit processeur étant en outre configuré pour, en exécutant la ou les instructions, sélectionner la dent cible prothétique en coupant les données de dent de préparation à l'aide de la ligne de marge.

14. Appareil de traitement de données de la revendication 9, ledit processeur étant en outre configuré pour, en exécutant la ou les instructions :
générer une surface externe d'un modèle de prothèse pour restaurer la dent cible prothétique à l'aide de la dent cible prothétique reconnue automatiquement à partir de la première image intrabuccale sur la base de la ligne de marge ;
générer une surface interne du modèle de prothèse à l'aide de la dent cible prothétique reconnue automatiquement à partir de la seconde image intrabuccale sur la base de la ligne de marge ; et
générer le modèle de prothèse à l'aide de la surface externe du modèle de prothèse et la surface interne du modèle de prothèse.

15. Support d'enregistrement non transitoire lisible par ordinateur sur lequel est stocké un programme qui, lorsqu'il est exécuté, amène un appareil informatique à effectuer des opérations, les opérations comprenant :
l'obtention d'une première image intrabuccale (10)
comprenant des données de dent de pré-préparation et d'une
seconde image intrabuccale (20) comprenant des données de dent de préparation ;
l'obtention d'une ligne de marge (600) sur la base des données de dent de préparation ; et
la reconnaissance et la sélection automatiques d'une dent cible prothétique à partir d'au moins l'une de la première image intrabuccale (10) et de la seconde image intrabuccale (20) sur la base de la ligne (600).
